# EUROPÄISCHE PATENTSCHRIFT

(11) **EP 3 096 126 B1**
(45) Veröffentlichungstag und Bekanntmachung des Hinweises auf die Patenterteilung: **20.02.2019**
(21) Anmeldenummer: 16169834.5
(22) Anmeldetag: 17.05.2016
(51) Int. Cl.: G01N 11/14, G01L 1/26

(54) **ROTATIONSRHEOMETER**
ROTATIONAL RHEOMETER
RHÉOMÈTRE ROTATIF

(30) Priorität: 20.05.2015 AT 504082015
(43) Veröffentlichungstag der Anmeldung: 23.11.2016
(73) Patentinhaber: Anton Paar GmbH, 8054 Graz-Straßgang (AT)
(72) Erfinder: KRENN, Michael, 8141 Zettling (AT); TRIEBL, Andreas, 8463 Leutschach (AT)
(74) Vertreter: Wildhack & Jellinek

(56) Entgegenhaltungen:
- WO-A1-2015/069633
- AT-B1- 508 706
- DE-A1- 4 112 371
- JP-A- H1 123 434
- Anonymous: "Tipps und Tricks von Joe Flow - Normalkraftregelung: Möge die Kraft mit dir sein" In: "Tipps und Tricks von Joe Flow - Normalkraftregelung: Möge die Kraft mit dir sein", 6 July 2018 (2018-07-06), Anton Paar, XP055493274,
- Anonymous: "Kraftfluss" In: "Kraftfluss", 6 July 2018 (2018-07-06), XP055493276,

## Beschreibung

Die Erfindung betrifft ein Rotationsrheometer gemäß dem Oberbegriff des Patentanspruchs 1.

Der Erfindung liegt vor allem die Aufgabe zugrunde, für Rheometer und somit auch für Viskosimeter, die keine eingebaute eigene Normalkraftmessung besitzen, eine Normalkraftbegrenzung zur Verfügung zu stellen, die den Messspalt konstant hält und Beschädigungen bei zu hohen Kräften verhindert. Viskosimeter sind im Fall der Erfindung Rheometern gleichgestellt.

Die Rheologie beschäftigt sich definitionsgemäß mit der Deformation und dem Fließverhalten von Substanzen. Rotationsrheometer sind Messgeräte zur Untersuchung der rheologischen Eigenschaften von unterschiedlichen Materialproben. Viskosimeter sind spezielle Rheometer zur Viskositätsbestimmung. Dabei wird die zu untersuchende Messprobe in einen im allgemeinen engen Messspalt zwischen zwei Messteilen eingebracht, die beiden Messteile werden relativ zueinander rotiert und/oder oszilliert und die Materialprobe wird zwischen den Messteilen einer Scherbelastung unterzogen. Elastische Materialfunktionen erhält man zusätzlich aus den Axialkräften, die beim Kegel-Platte- und Platte-Platte-Rheometer o.ä. Anordnungen senkrecht zur Scherebene wirken. Dabei ist eine Vielzahl weiterer möglicher Messteilgeometrien bekannt. Rotationsrheometer ermöglichen die Kombination einer Vielzahl unterschiedlicher Versuchsführungen, bei denen entweder Schubspannung, Scherdeformation oder Schergeschwindigkeit vorgegeben werden können. Rotationsrheometer können prinzipiell unterschiedliche Ausführungen mit einem Messmotor, einem Rotationsmotor und separatem Messmotor oder die Kombination zweier Messmotoren besitzen und sind beispielsweise in der AT508706 B1 beschrieben.

Die Drehmomentbestimmung im Rotationsrheometer kann mit für Antrieb und Drehmomentbestimmung ausgelegten (Mess-)Motoren erfolgen oder auch über zwei voneinander getrennte Motoreinheiten für Antrieb/Rotation und Drehmomentbestimmung, die jeweils einem der Messteile zugeordnet werden. Je nach Gerätetyp rotiert dabei der obere Messteil, , oder der untere Messteil, beispielsweise ein Messbecher. Es werden das resultierende Drehmoment und/oder die Phasenlage bestimmt. Ferner ist auch ein Doppelmotorsystem mit zwei Messmotoren bekannt, z.B. AT 508 706 B1. Darüber hinaus sind unterschiedliche Systeme zur Bestimmung der auftretenden Normalkräfte (Axialkräfte) bekannt.

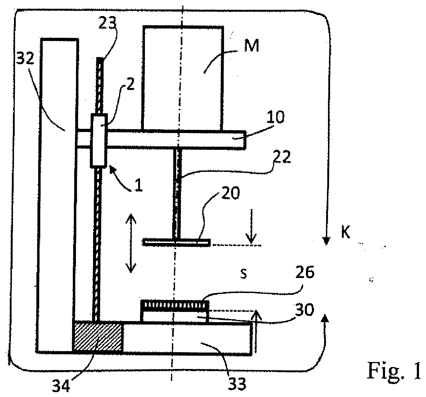

In der Fig. 1 ist eine bekannte Ausführungsform eines Drehrheometers dargestellt. Die zu vermessende Mediumsprobe befindet sich auf dem unteren Messteil 30 (hier eine Platte). Durch Absenken des oberen Messteils 20 in Kontakt mit der zu untersuchenden Probe wird der Messspalt S gebildet.

Das hier exemplarisch gezeigte Rheometer verfügt über einen Messmotor M, der die Messwelle 22 und den mit dieser verbundenen oberen Messteil 20 rotiert bzw. rotierend oszillierend antreibt. Dabei ist der Zusammenhang zwischen dem Drehmoment an der Messwelle 22 und der Leistungsaufnahme des Messmotors M genau bekannt oder kann durch Kalibrierung ermittelt werden. Unterschiedliche Messsysteme und möglichst reibungsfreie Lageranordnungen ermöglicht die Bestimmung von Drehwinkel und Rotationsgeschwindigkeit. Alle Werte stehen in einer Auswerteeinheit, die nicht dargestellt ist, zur Verfügung.

Die rheologischen Kenngrößen der Mediumsprobe werden in der Auswerteeinheit aus dem Drehmoment bzw. aus den Versorgungsparametern, insbesondere aus der Stromaufnahme des Messmotors M und/oder aus der Frequenz und/oder aus der Phasenlage der Messwelle 22 und den Spaltdaten unter Berücksichtigung von Messgeometriedaten der Messteile und der Temperatur, ermittelt.

Die für die Messgenauigkeit im Rheometer bzw. Viskosimeter bestimmenden Faktoren sind neben der genauen Bestimmung des Drehmoments und der Stabilität der Umgebungsbedingungen die möglichst reibungsfreie Lagerung der Antriebs- und/oder Messwellen und insbesondere auch die Steifigkeit des Stativs. Um das jeweilige momentan wirkende Drehmoment mit minimalen Reibungs- und Zugkräften zu messen, sind beispielsweise Magnet- und Luftlagerungen für die rotierten Komponenten bekannt.

Von großem Einfluss auf die Messgenauigkeit ist dabei auch der tatsächliche Abstand der beiden Messteile zueinander bzw. die Höhe bzw. Dicke des Spalts S. Der Messmotor und die Messteile liegen dabei in verstellbarem und während der Messung in konstant gehaltenem axialem Abstand zueinander an einem Stativ 32 bzw. Gestell 33. Dabei wird zumindest einer der Messteile von einer gegebenenfalls automatischen Stelleinheit betätigt bzw. von Stellteilen verstellbar getragen.

In Fig. 1 ist eine konventionelle Art der Verstellung der Spalthöhe S dargestellt: Ein am Stativ 32 montierter Antrieb bzw. Vorschubmotor 34 verschiebt den von dem Motor M am oberen Stellteil bzw. Träger 10 getragenen Messteil 20 mittels der von einer Schraubspindel 23 und Spindelmutter 2 gebildeten Verstelleinheit 1 relativ gegenüber dem unteren Messteil 30, der von einer Grundplatte 33 getragen wird. Die zu untersuchende Probe wird 26 wird zwischen die beiden Messteile 20, 30 eingebracht und nach der Spalteinstellung gegebenenfalls getrimmt. Zur Spalthöhen-Einstellung erfolgt die Regelung des Vorschubmotors der Spindel 23 mittels der von einem berührungslos arbeitenden, an der Spindel 23 angeordneten Abstand- bzw. Weg- bzw. Längenmesseinheit gemessen Werte. An sich bekannte Längenmesseinheiten, wie z.B. Potentiometer, inkrementale Wegaufnehmer, induktive Messaufnehmer oder Messuhren, messen jeweils die Distanz zwischen den Messteilen 20, 30 und/oder zwischen der Spindelmutter 2 und einem Fixpunkt am Stativ 32 bzw. an der Grundplatte 33 oder anderen Fixpunkten. Die Messung des tatsächlichen Abstands s kann sowohl absolut durch entsprechende Einjustierung für die jeweils verwendete Messgeometrie am Höhen-Vorschub- und -Verstellsystem bzw. an der Stelleinheit erfolgen als auch relativ, d.h. ausgehend vom jeweils durch geeignete Verfahren, beispielsweise durch den Drehmomentanstieg oder Normalkraftanstieg beim Einander-Berühren der Messteile ermittelten Spalt-Nullpunkt. Auch der Einsatz von Steppermotoren, um eine gesteuerte Positionierung der Stellteile relativ zueinander, ohne die Notwendigkeit eine AbstandsMessung durchzuführen, ist bekannt.

Unter konstanten Umgebungsbedingungen können damit vorgegebene Spalthöhen S mikrometer-genau angefahren werden und gleichzeitig der benötigte Vorschub im mm- bzw. cm-Bereich für das Einbringen des Probenmediums in den Messspalt bzw. für das Wechseln der Messteile erzielt werden.

Aber auch jeder anders ausgebildete präzise mechanische Linearvorschub kann verwendet werden, wie beispielsweise Linear-Motoren, pneumatisch angetriebene Verstelleinrichtungen, Uhnig-Mutter-Antriebe u. dgl., um durch Absenken und Anheben des unteren Messteils 30 und/oder des oberen Messteils 20 die Probe in den Messspalt S einbringen zu können und den gewünschten Messspalt zwischen den Messteilen 20, 30 herzustellen.

Bei einem Rheometer mit Stativ und vor allem bei automatisierten Spalt-Systemen besteht das Problem, dass beim Eintauchen des Messsystems in die Probe und beim Ausfahren des Messsystems aus der Probe unterschiedliche Normalkräfte auf das Lager des Messmotors wirken. Diese Kräfte hängen von der Viskosität der Probe ebenso ab, wie von der Art des Messsystems und der Verfahrgeschwindigkeit. Bei einer vom Benutzer falsch gewählten Verfahrgeschwindigkeit kann es zum Auslösen eines mechanischen Überlastschalters, zur Blockierung des Antriebs oder sogar zur Beschädigung der Messvorrichtung kommen. In allen diesen Fällen wird der Messspalt S verändert und die Probe muss aus dem Messgerät genommen und dieses neu referenziert werden.

In vielen Fällen besitzen Rheometer Normalkraftmesseinrichtungen, die es ermöglichen, nicht nur die wirkenden Drehmomente zu untersuchen, die aufgrund der Probenviskosität der Scherbelastung entgegen wirken, sondern auch die senkrecht zur Scherbelastung wirkenden Normalkräfte zu messen. Diese Kräfte treten aufgrund der elastischen Anteile von realen viskoelastischen Proben praktisch immer auf. Unterschiedlichste Varianten zur Normalkraftmessung sind im Stand der Technik bekannt, beispielsweise im Luftlager eines Rotationsrheometers befindliche, berührungslos arbeitende Normalkraftsensoren.

Kostengünstige Viskosimeter bzw. Rheometer, beispielsweise zur Überwachung und Standardcharakterisierung von Proben in Produktionsprozessen, werden aber oftmals aus Kostengründen ohne Normalkraftmessung ausgeführt. In dieser Genauigkeitsklasse werden die Messantriebe z.B. mit Kugellagerung oder günstigen Luftlagern ausgeführt.

Ziel der Erfindung ist es, ein System zu entwickeln, das Normalkräfte in positiver und/oder negativer Richtung bis zu einer maximalen Grenzkraft ohne nennenswerte bzw. zu berücksichtigende Bewegung der Messteile und somit ohne wesentliche Messspaltänderung aufnehmen kann, das Rheometer also völlig steif hält und beim Überschreiten dieser Grenzkraft aber unmittelbar einen Kontakt öffnet und somit das Auftreten einer die Grenzkraft überschreitenden Normalkraft detektierbar macht. Dadurch kann vom Rheometer die Verfahrbewegung bis zum Absinken der Normalkraft unter den Grenzwert gestoppt werden. Sinkt die Normalkraft wieder unter den Grenzwert, schließt sich der Kontakt und die mechanische Position ist wieder absolut identisch wie vor dem Öffnen.

Da ein Schlüsselelement für die genaue Charakterisierung der Probe die Konstanz des Messspalts ist, müssen alle Sicherheitseinrichtungen praktisch wegfrei ausgestaltet werden, der Messspalt zwischen den Messteilen muss konstant gehalten werden.

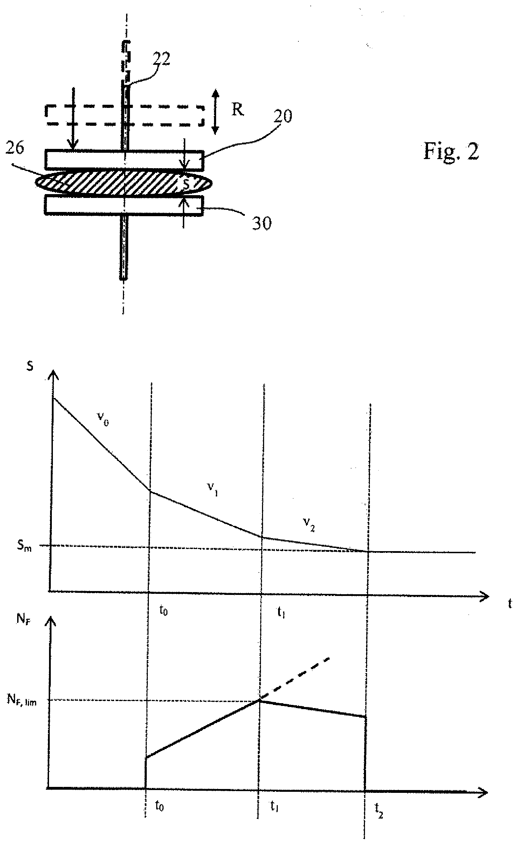

Beispielsweise dient der beim Erreichen des Berührungspunkts des oberen Messteils mit der Probe auftretende Normalkraftsprung zur Feststellung, wann die beiden Messteile 20, 30 beim Zusammenfahren mit der Probe in Kontakt getreten sind und damit das Annähern der beiden Messteile verlangsamt werden muss. Speziell viskoelastische Proben mit hohem elastischen Anteil wie z.B. Polymerschmelzen, Asphalt, Schokolade etc., werden erst zwischen den beiden Messteilen 20, 30 in Form gequetscht und nach Einstellung der erwünschten Messspaltweite werden gegebenenfalls überstehende Probenränder getrimmt. Verfügt das Rheometer über eine Normalkraftmesseinheit, wird mittels dieser sichergestellt, dass die empfindlichen Bauteile, insbesondere Lager etc., des Rheometers nicht beschädigt werden. Der in Fig. 2 dargestellte prinzipielle Verlauf der auftretenden Normalkräfte während der Annäherung kann direkt durch die Steuer- und Regeleinheit zur Regelung der Motorgeschwindigkeit des Verstellmechanismus verwendet werden. Die Annäherung erfolgt bis zum Berühren mit maximaler Geschwindigkeit, ab dem Berühren bewegen sich die Messteile nur mehr langsam aufeinander zu. Dabei kann die maximal zulässige Normalkraft, die auftreten darf, in der Steuer- und Auswerteeinheit als Schwellwert hinterlegt sein.

In Fig. 2 ist die Entwicklung der Normalkraft N_{F} und des Abstands S zwischen den beiden Messteilen 20, 30 bei Annäherung bzw. Zusammenfahren der Messteile schematisch über die Zeit t dargestellt

Die beiden Messteile 20, 30 des Rheometers, hier ein Platte-Platte-Messsystem, werden in der Richtung R aneinander angenähert. Der Abstand zwischen den beiden Messteilen 20, 30 wird zu Versuchsbeginn so gewählt, dass die Probe zwischen die Messteile gut eingebracht werden kann und daher ist der Abstand zwischen den Messteilen groß. In den schematischen Diagrammen ist die Entwicklung der Spaltweite des Messspalts S zwischen den Platten und die gemessene bzw. auftretende Normalkraft zwischen den Messteilen 20, 30 dargestellt. Der erste Abschnitt zeigt eine rasche Annäherung der beiden Messteile bis zum Zeitpunkt t₀ - hier steigt die Normalkraft sprunghaft an. Ab diesem Zeitpunkt ist die Probenoberfläche mit beiden Messteilen in Kontakt. Die weitere Annäherung folgt im Regelfall nur mehr langsamer, um die Probe gleichmäßig zwischen den Messteilen einzubringen. Wird zu einem Zeitpunkt (hier t₁) die höchste zulässige Normalkraft überschritten, dann kann über die Steuer- und Regeleinheit bzw. die Verstelleinrichtung die Verstellgeschwindigkeit weiter reduziert werden. Die Annäherung erfolgt mit v3 oder wird gestoppt, um ein Angleichen der Probe an die Spaltweite zu ermöglichen. Bei Erreichen der gewünschten (Mess-)Spaltweite sₘ kann die Probe ggf. getrimmt werden und die Messung starten. Die gezeichneten Normalkraftverläufe können natürlich in Abhängigkeit der Probe auch nicht linear verlaufen und sind hier nur beispielhaft dargestellt.

Ein weiterer Problemfall ist auch die Begrenzung der auftretenden Kräfte beim Auseinanderfahren der beiden Messteile nach der Versuchsdurchführung:

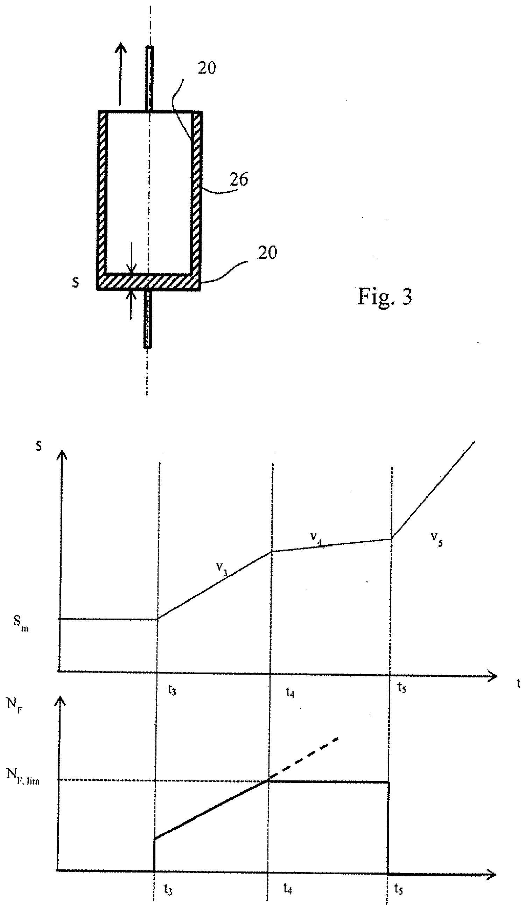

Fig. 3 zeigt eine derartige Anordnung mit einem Zylindermesssystem mit zwei konzentrisch angeordneten, zylindrischen Messteilen 20, 30, die nach der Messung auseinandergefahren werden. Die Entwicklung der Normalkraft N_{F} und des Abstands zwischen den beiden Messteilen 20, 30 ist schematisch über die Zeit t dargestellt.

Viele untersuchte Proben, z.B. nach dem Aushärten von Gummi, Verfestigen von Schokolade etc., kleben an den Messteilen fest. Insbesondere bei Verwendung von Zylindermesssystemen mit naturgemäß großen Kontaktflächen zwischen Messteiloberfläche und Probe kann das Messsystem beschädigt werden. Bei Vorliegen einer Normalkraftmessung verringert das System aufgrund der auftretenden negativen Normalkräfte die Verfahrgeschwindigkeit des Trägers des Messteils.

Nach Ende der Messung mit einem definierten Abstand der Messteile Sₘ startet zum Zeitpunkt t₃ das Lösen der Messteile von der Probe durch Verfahren mit der Geschwindigkeit v₃. Sollten die dabei auftretenden Normalkräfte durch Effekte wie z.B. Kleben ansteigen und die eingestellte Grenzkraft überschreiten (Zeitpunkt t₄), wird die Geschwindigkeit des Antriebs angepasst (v₄) oder dieser gestoppt. Wenn sich die Probe vollständig von einem der Messteile gelöst hat oder der Kontakt mit den auf beiden Messteilen haftenden Probenresten unterbrochen ist, kann mit maximaler Geschwindigkeit weiter verfahren werden. Zum Zeitpunkt t5 sinkt N_{F} auf 0 ab.

Auch hier sind die Verläufe des Abstands zwischen den Messteilen 20, 30 und die Entwicklung der Normalkraft N_{F} schematisch dargestellt. Tatsächliche Verläufe zeigen in Abhängigkeit von den Probeneigenschaften unterschiedliche funktionale Abhängigkeiten.

Ein weiteres Problem sind die bei manchen Proben auftretenden extrem hohen auftretenden Normalkräfte, die während einer Versuchsdurchführung, z.B. Temperierung der Probe, Scherverdickung od. dgl., auftreten können und eine sofortige Unterbrechung der Messung erfordern.

Hat ein kostengünstiges Rheometer bzw. Viskometer, z.B. für die Standardcharakterisierung, keine Normalkraftmessung implementiert, kann ein abgesehen von den empfindlichen Mess- und Lagerteilen auch allenfalls verwendetes automatisches bzw. motorgesteuertes Stativ durch die auftretenden Überlasten beschädigt werden, wenn der Schrittmotor durch die Motorsteuerung nicht gedrosselt bzw. abgestellt werden kann.

Bis jetzt wurde dieses Problem dadurch gelöst, dass extrem langsame Geschwindigkeiten für das automatische Verfahren mit dem Stativ gewählt werden mussten und daher auch bei Qualitätsmessungen unnötiger Zeitverlust in Kauf genommen werden müsste.

Selbst bei händischem Verstellen am Stativ kann hier das empfindliche Motorlager Schaden nehmen, wenn trotz hoher auftretender Kräfte der Verstellmechanismus des Stativs weiterbewegt wird.

Wenn hier eine exakte Kraftmessung oder ein Begrenzerschalter eingebaut würde, darf der Messspalt nicht beeinflusst werden. Speziell beim Annähern der Messteile zueinander darf der Schaltmechanismus keinerlei Verzögerung beim Auslösen erzeugen, da andernfalls die Spaltgeometrie geändert werden könnte.

Herkömmliche Schalter bedingen wegen der Hysterese, die jede Schaltung beim Auslöseverhalten hat, eine Änderung des Spalts durch die auftretende Normalkraft. Der Spalt darf sich aber speziell beim Annähern der Messteile zueinander abhängig von den auftretenden Kräften nicht ändern.

Erfindungsgemäß werden diese Probleme bei einem Rotationsrheometer der eingangs genannten Art mit den im Kennzeichnen des Anspruchs 1 angeführten Merkmalen gelöst.

Für eine exakte Schaltung ist es von Vorteil, wenn die Kontaktteile des Schaltkontakts in einer Richtung relativ zueinander bewegbar angeordnet sind, die parallel zur Richtung der relativen Bewegung der Messteile im Zuge ihrer Verstellung verläuft oder eine parallel zu dieser Bewegungsrichtung verlaufende Komponente besitzt.

Ein mechanisch vorteilhafter Aufbau ergibt sich, wenn die beiden Kontaktteile des jeweiligen Schaltkontakts jeweils auf einem von zwei relativ zueinander bewegbaren bzw. voneinander baulich eigenständigen bzw. trennbaren Bauteilen des Kraftkreises des Rotationsrheometers liegen und dass zumindest eine Federeinheit vorgesehen ist, mit der diese beiden Bauteile und die Kontakte des Schaltkontakts durch Federkraft aneinander oder aufeinander zu gedrückt und in dieser Lage gehalten sind.

Je nach Einsatzzweck des Rheometers ist es möglich, dass der Schaltkontakt auf einzelnen Komponenten des Kraftkreises des Rotationsrheometers, vorzugsweise einem Träger oder einer Tragplatte des Mess- bzw. Antriebsmotors, im Verlauf einer Messwelle, im Verlauf einer Antriebsspindel der Verstelleinrichtung oder im Verlauf der Halterung für den Mess- oder Antriebsmotor oder jeweils zwischen den einzelnen Komponenten oder im Verbindungs- bzw. Übergangsbereich von einer Komponente zur anderen angeordnet sind.

Für den Aufbau des Rotationsrheometers ist es zweckmäßig, wenn der Kraftkreis des Rotationsrheometers als Komponenten ein Stativ, ein Gestell, einen Träger für den Antriebs- oder Messmotor sowie den Messteil und die Messwelle, und die Verstelleinrichtung zur Höhenverstellung des Messteils bzw. die Spindel und/oder deren Antrieb umfasst.

Ein einfacher und genauer Aufbau ergibt sich, wenn die für die Abschaltung oder Stillsetzung der Verstelleinrichtung zusammenwirkenden, gegebenenfalls als Kontaktflächen ausgebildeten, Kontaktteile des Schaltkontakts auf physisch trennbaren und unabhängig voneinander bewegbaren Bauteilen bzw. Komponenten des Kraftkreises liegen, welche in der Richtung, in der der Messspalt veränderlich und verstellbar ist bzw. in der die Messteile aufeinander zu bzw. voneinander weg bewegbar sind bzw. insbesondere parallel zu der in der Probe auftretenden und gegebenenfalls gemessenen Komponente der Normalkraft, mit Federkraft gegeneinander gedrückt sind.

Zur Schadensvermeidung ist es von Vorteil, wenn die Verstelleinrichtung bei Öffnung des Schaltkontakts die Verstellbewegung der Messteile relativ zueinander abrupt abbricht und/oder wenn der Träger zumindest mit zwei Bauteilen gebildet ist, wobei die jeweiligen im Kraftkreis aufeinanderfolgenden Bauteile jeweils mit zumindest einer Federeinheit mit vorgegebener Federkraft aneinander gedrückt sind und dass bei Einwirken einer die Federkraft übersteigenden Trennkraft auf die Bauteile die beiden aneinander angedrückten Bauteile voneinander entfernbar sind und gleichzeitig damit die Kontaktteile des Schaltkontakts voneinander trennbar sind.

Ein für die Praxis vorteilhaft einsetzbares und einfach aufgebautes Rheometer mit einer auf der Antriebsspindel höhenverstellbar gelagerten Mutter ergibt sich, wenn die Mutter einen, vorzugsweise ringförmigen, Kontaktbauteil mit einem nach oben ragenden Kontaktteil trägt, wobei oberhalb des Kontaktteils ein vorzugsweise von einem Kontaktring gebildeter Kontaktteil liegt, der mit einer Spannhülse verbunden ist, die in ihrem unteren Ende einen Spannring trägt, wobei zwischen dem Spannring und dem Träger des Antriebs- oder Messmotors bzw. der Messwelle eine Schraubenfeder mit vorgegebener Federkraft angeordnet ist und zwischen der Mutter und dem Spannring eine weitere Schraubenfeder mit vorgegebener Federkraft angeordnet ist und/oder wenn der Träger einen nach oben ragenden Kontaktteil trägt, dass oberhalb des Trägers ein Kontaktbauteil mit einem nach unten ragenden Kontaktteil angeordnet ist, der von oben her an den vom Träger getragenen Kontaktteil anlegbar ist, welcher Kontaktteil von einer Spannhülse getragen ist, die in ihrem unteren Ende einen Spannring trägt, wobei zwischen dem Spannring und dem Träger des Antriebs- oder Messmotors bzw. der Messwelle eine Schraubenfeder mit vorgegebener Federkraft angeordnet ist und zwischen der Mutter und dem Spannring eine weitere Schraubenfeder mit vorgegebener Federkraft angeordnet ist.

Ein für die Geometrie des Rheometers vorteilhafter Aufbau ergibt sich, die Schraubenfeder die weitere Schraubenfeder umgibt und gegebenenfalls unterhalb des Schaltkontakts verläuft und/oder dass die Spannhülse zwischen der Schraubenfeder und der weiteren Schraubenfeder liegt und/oder dass die weitere Schraubenfeder die Spindel umgibt und/oder wenn die Kontaktteile mit Blatt- oder Schrauben- oder Spiralfedern belastet sind, deren Federkraft in Richtung der Öffnungs- und Schließbewegung der Kontaktteile wirkt oder eine in dieser Richtung wirkende Federkraftkomponente besitzt.

Es ist für eine Praxis durchaus zweckmäßig, wenn zur Detektion der von zumindest einem Messteil im Zuge seiner Verstellung durch eine von der Probe ausgeübte Kraft zusätzlich zu den auf den Kraftfluss im Kraftkreis ansprechenden Schalteinheit bzw. Schaltkontakten eine Einrichtung zur Messung der auftretenden, von der Probe ausgeübten positiv oder negativ zur Richtung des Kraftflusses gerichteten Normalkraft F_{N} vorgesehen ist.

Für die Führung der Messverfahren ist es vorteilhaft, wenn die Hubfeder und die Senkfeder jeweils mit einer Vorspannungskraft bzw. einem Grenzkraftwert vorgespannt sind, die bzw. der dem Gewicht der zu tragenden Komponenten bzw. Apparatur des Rotationsrheometers und einem vorgegebenen Kraftwert entspricht, wobei dieser vorgegebene Kraftwert einem von der Probe aus auf den Messteil in positiver und negativer Richtung des Kraftflusses ausgeübten und noch als tolerierbar angesehenen Kraftwert entspricht.

Erfindungsgemäß kann die Schalteinheit ein ein- oder doppelseitig gegen einen Anschlag vorgespanntes Federsystem mit Schaltkontakten aufweisen. Damit erfolgt bis zur Auslösung durch Überschreiten des Grenzkraftwerts keine Bewegung im Detektionssystem. Bei einer Überschreitung des Grenzkraftwerts wird der Schaltkontakt, der elektrisch über die Kontaktflächen der vorgespannten Federeinheiten realisiert werden kann, durch eine Minimalauslenkung im µm-Bereich unterbrochen.

Durch Anbringung der Federeinheiten, z.B. Spiralfedern, Blattfedern o.ä., kann der elektrische Schaltkontakt entgegen dem Grenzkraftwert bzw. der maximal zulässigen Kraft N_{F} vorgespannt werden. Die dafür angeordnete(n) Feder(n) drücken die vorgespannten Komponenten bzw. Bauteile des Rheometers entlang des Kraftkreises aneinander und stellen dort an den einander berührenden Kontaktflächen einen elektrischen Kontakt her. Übersteigt die auftretende Kraft das durch die Steifigkeit der Federn implementierte Limit, so wird der Kontakt unterbrochen und dieser unterbricht die Bewegung der Verschiebeeinheit, beispielsweise die Stromversorgung des Schrittmotors.

Dieser vorgespannte Schaltkontakt kann an unterschiedlichen Stellen im Kraftkreis des Rheometers eingebaut werden. Dabei können die beiden Kontaktteile des Schaltkontakts beliebig separat im Kraftkreis des Rheometers eingebaut werden, oder aber die Kontaktteile werden bevorzugt symmetrisch bzgl. der Spindel oder der Messwelle gemeinsam mit den Federelementen im Rheometer eingebaut.

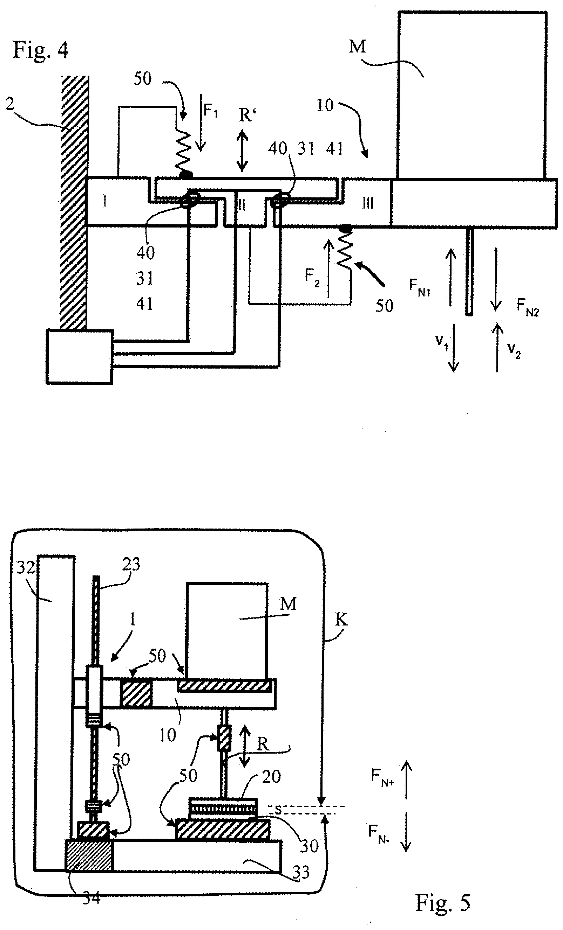

Figur 4 zeigt exemplarisch eine Ausführungsvariante der Erfindung im schematischen Schnitt durch den Tragarm bzw. Träger 10 des Rheometers mit einer in beide Verstellrichtungen, d.h. nach oben und unten wirkenden Federeinheiten. Der Tragarm bzw. Träger 10 ist insgesamt in drei Segmente bzw. Bauteile I, II, III geteilt. Die Segmente I, II, III werden durch die Federkraft der vorspannenden Federeinheiten 50 fest aneinandergedrückt, die Gesamt-Steifigkeit des Rheometers bleibt somit bis zur Überschreitung des Grenzkraftwerts unbeeinflusst.

Eine Federeinheit 50 drückt das als T-förmiger Aufleger ausgebildete Segment II mit der Kraft F1 gegen den linken Tragarmteil I, ein zweites Federelement 50 drückt das Segment III mit der Kraft F2 gegen den Tragarmteil bzw. das Segment II. Gleichzeitig werden die Kontaktteile 31, 41 der Schaltkontakte 40 aneinander gepresst.

Bei beiden Schaltkontakten 40 werden die elektrischen Kontaktteile 31, 41 durch die Federkraft geschlossen gehalten und der Stromkreis bzw. Versorgungskreis des Verschiebemechanismus der Verstelleinrichtung 1 ist mit diesen Schaltkontakten schaltbar.

Wird nun der Tragarm bzw. Träger 10 mit dem oberen Messteil 20 und dem Messmotor M mit der Geschwindigkeit v1 auf den unteren Messteil 30 zu bewegt, dann treten ab dem Kraftschluss mit der Probe, da die Probe mit beiden Messteilen 20, 30 Kontakt hat, Normalkräfte F_{N1} entgegen der Bewegungsrichtung R' auf. Bis zum Erreichen der Auslösekraft bzw. des Grenzkraftwerts F_{N1} = -F1 bleiben das Stativ 32 und das Gestell 33 und die Verschiebeeinrichtung 1 von den auftretenden Kräften unbeeinflusst.

Wird der Grenzkraftwert erreicht, wird die Federkraft egalisiert und es heben die Kontaktflächen der Kontaktteile 31, 41 voneinander ab und die Tätigkeit der Verschiebeeinrichtung 1 wird bei geringstem möglichen Verschiebeweg und ohne Zeitverzögerung oder Hystere durch Öffnung die Schalteinheit 40 unterbrochen.

Gleiches gilt für die Bewegung des Trägers 10 in Gegenrichtung mit der Geschwindigkeit v2 und den auftretenden Kräften F_{N2} in der Gegenrichtung.

Bis zum Erreichen der Auslösekraft bzw. Grenzkraft F_{N2} = -F2 bleiben das Stativ 32 und das Gestell 33 und die Verschiebeeinrichtung 1 von den auftretenden Kräften unbeeinflusst; die federbelasteten Kontaktteile 31, 41 bleiben aneinandergepresst.

Es ist möglich, den Tragarm bzw. Träger 10 auch nur einmal zu teilen. Entsprechend ist dann nur ein Abschalten der Verstelleinheit bei Auftreten der Normalkraft F_{N1} oder F_{N2} möglich.

Um das Erfindungsprinzip umsetzen zu können, kann die hier schematisch dargestellte Federeinheit 50 mit beliebiger Federgeometrie realisiert werden, z.B. können Blattfedern oder Schraubenfedern, Verwendung finden.

Damit ist ein Prinzip zur Normalkraftbegrenzung ohne explizite bzw. tatsächliche Messung der Normalkraft realisiert. Diese rein mechanische Aufbauvariante ist robust, kostengünstig und betriebssicher.

Im Sinne der Erfindung sind unter einer Federeinheit 50 alle Elemente zu verstehen, die die Kontaktflächen 31, 41 der Schaltkontakte 40 mit definierter Vorspannung gegeneinander pressen können. Neben mechanischen Federsystemen können auch als alternative Umsetzungsvarianten Pneumatikzylinder und/oder elektromagnetische Federn und/oder Permanentmagnete gewählt werden.

Fig. 5 zeigt schematisch eine Rheometeranordnung mit einem Stativ 32 und einem Gestell 33 sowie mit einem Messmotor M, der über den Tragarm bzw. Träger 10 mit dem Stativ 32 verbunden auf diesem höhenverstellbar gelagert ist.

Exemplarisch sind hier unterschiedliche Einbauvarianten der Schalteinheit und der Federeinheiten 50 in den Kraftkreis K des Rheometers dargestellt. Ist der Messspalt S geschlossen und sind beide Messteile 20, 30 in Kontakt mit der Probe, ist der prinzipiell dargestellte Kraftkreis K geschlossen. Das Stativ 32 und der Messmotor M werden möglichst steif ausgeführt, damit durch die an den kraftführenden Komponenten auftretenden Kräfte der Messspalt S nicht verändert wird. Die erfindungsgemäß vorgesehenen Federeinheiten 50 können an unterschiedlichen Stellen in diesen Kraftkreis K eingebaut werden. In Fig. 5 sind einige in Frage kommende Stellen schematisch durch die schraffierten Flächen dargestellt, nämlich direkt im Bereich des Stativs 32, linear im Tragarm bzw. Träger 10 oder rotationssymmetrisch um die Motorauflage M', oder nahe an den beiden Messteilen 20, 30. Wird eine Federeinheit 50 in die Messwelle 22 eingebaut, muss darauf geachtet werden, dass die Drehmomentmessung nicht durch das Federsystem beeinflusst wird, die Ausführung sollte rotationssymmetrisch sein, was auch für einen Einbau an der Spindel 23 zutrifft.

Wird bloß händisch verstellt, so kann anstelle einer Stillsetzung der Verstelleinheit mit der Schalteinheit ein Warnsignal abgegeben werden und das händische Verstellen wird beendet.

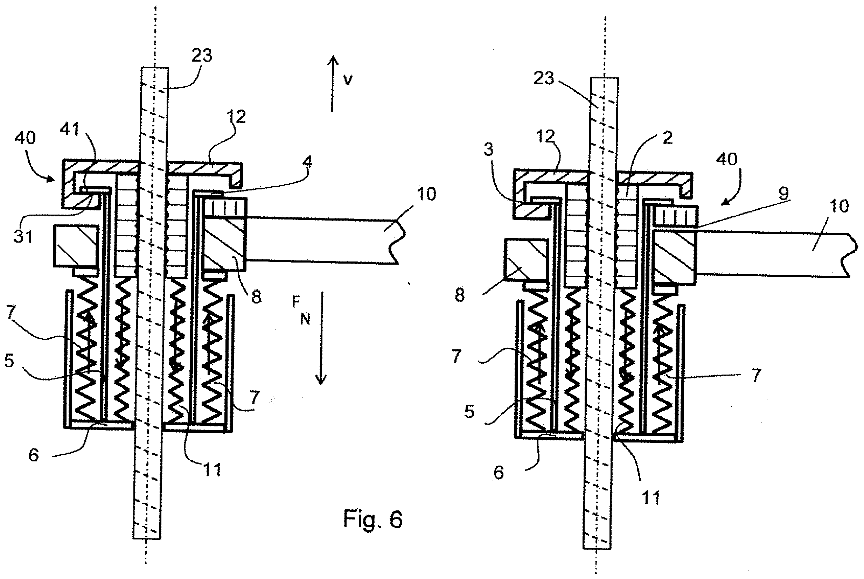

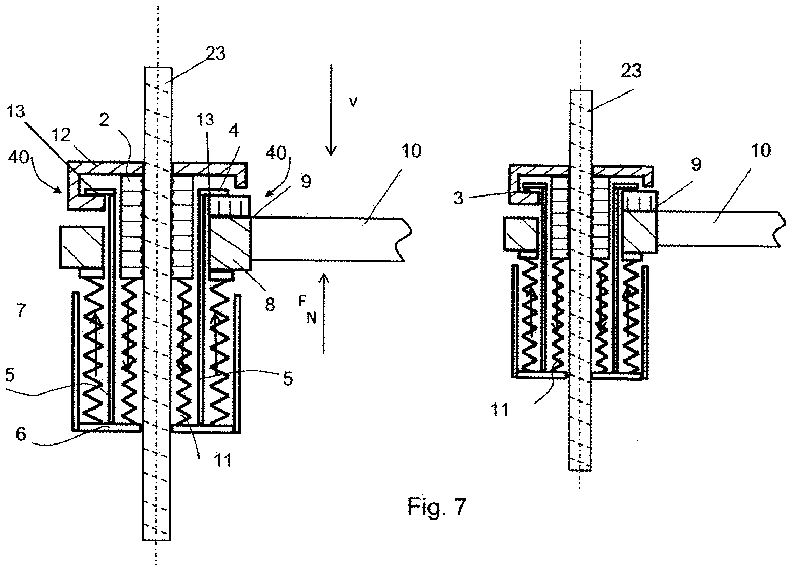

Bevorzugt wird die Normalkraftbegrenzung für beide Bewegungsrichtungen R in einem kombinierten Bauelement realisiert, bei dem zwei Federeinheiten 50 gegeneinander wirken und die Kontaktflächen 31, 41 gegen beide Kraftrichtungen R' vorspannen, wie in Fig. 6 und 7 dargestellt ist. Natürlich kann die Normalkraftbegrenzung auch nur in einer Wirkungsrichtung ausgeführt werden oder die beiden Wirkungsrichtungen können getrennt voneinander im Kraftkreis K des Rheometers realisiert werden.

Eine bevorzugte Ausführungsvariante für den Einbau einer Schalteinheit bzw. von Schaltkontakten 40 in die Spindel 23 eines Linearatriebs für den Träger 10 ist in Fig. 6 und 7 dargestellt.

Die Drehbewegung der Spindel 23 wird über die Mutter 2 in eine vertikale Bewegung mit der Geschwindigkeit v übergeführt, wobei das Verdrehen der Mutter 2 gegebenenfalls durch einen Überlaststift, der beispielsweise in einem Mitnehmerbacken oder Haltering 8 geführt wird, verhindert wird.

Bewegt sich die Mutter 2 gemäß Fig. 6 nach oben, so wirkt die Hubkraft von der Mutter 2 über den Fixierring bzw. ringförmigen Kontaktbauteil 12, die Kontaktfläche 31 des NF-minus Kontakts 3, den Kontaktring 4 mit der Kontaktfläche 41, die Spannhülse 5 und den Spannring 6 auf eine Hubfeder 7, z.B. Schraubenfeder, die sich über den Mitnehmerbacken bzw. Haltering 8 an der Mitnehmerplatte bzw. dem Träger 10 abstützt. Wird der Träger 10 gegen eine vertikale Bewegung nach oben gesperrt und übersteigt die Hubkraft der Mutter 2 die Vorspannkraft der Hubfeder 7 minus dem Eigengewicht der zu hebenden Apparatur, d.h. Träger 10 mit Motor M sowie Messwelle 22 und oberer Messteil 20 und allfälligen weiteren, mitgetragenen Bauelementen, öffnet sich der NF-plus Kontakt 9.

Der Bewegung der Mutter 2 nach oben wirkt eine Normalkraft -FN entgegen. Diese Kraftwirkung setzt sich über den Träger 10 und den Haltering 8 auf die Hubfeder 7 fort, die zwischen dem Spannring 6 und der Mutter 2 wirkt. Mit Öffnen des NF-plus Kontakts 9 endet die Verstellbewegung des Trägers 10 abrupt. Links in Fig. 6 ist die Betriebsstellung dargestellt und rechts die Stellung, in der die Verschiebeeinheit 1 stillgesetzt ist.

Bewegt sich die Mutter 2 gemäß Fig. 7 nach unten, so wirkt die Senkkraft der Mutter 2 auf die Senkfeder 11, die sich über den Spannring 6, die Spannhülse 5, den Kontaktring 4, den NF-plus Kontakt 9 über den Mitnehmerbacken 8 an dem Träger 10 abstützt. Wird der Träger 10 gegen eine vertikale Bewegung nach unten gesperrt und übersteigt die Senkkraft der Mutter 2 die Vorspannkraft der Senkfeder 11 plus dem Eigengewicht der zu hebenden Apparatur um einen vorgegebenen Kraftwert, öffnet der NF-minus Kontakt 3.

Der Bewegung der Mutter 2 nach unten wirkt eine Normalkraft F_{N} entgegen. Diese Kraftwirkung setzt sich über den Träger 10, den Mitnehmerbacken 8, den NF-plus Kontakt 9, die Kontaktfläche 13, die Spannhülse 5 und den Spannring 6 fort, und wirkt der vorgegebenen Vorspannkraft der Senkfeder 11 entgegen.

Der Kraftfluss kann sich auch verändern, ohne dass die Messteile 20, 30 eine Bewegung ausführen.

## Patentansprüche

1. Rotationsrheometer mit zwei den Messspalt (S) begrenzenden, mit einer Verstelleinrichtung (1) in einer vorgegebenen Bewegungsrichtung (R) relativ zueinander bewegbaren Messteilen (20, 30),
**dadurch gekennzeichnet, dass** zumindest eine auf Änderungen im Kraftfluss in dem von den Messteilen (20, 30) begrenzten Kraftkreis (K) des Rotationsrheometers ansprechende Schalteinheit mit Kontaktteile (31, 41) aufweisenden Schaltkontakten (40) für die Tätigkeit der Verstelleinrichtung (1) vorgesehen ist, die die Verstelleinrichtung (1) bei Überschreiten eines vorgegebenen Grenzkraftwerts für den Kraftfluss in positiver Richtung, gegebenenfalls auch ohne Relativbewegung der Messteile bzw. bei lageinvariant verbleibenden Messteilen (20, 30), stillsetzt, und die die Verstelleinrichtung (1) bei Überschreiten eines vorgegebenen Grenzkraftwerts für den Kraftfluss in negativer Richtung, gegebenenfalls auch ohne Relativbewegung der Messteile bzw. bei lageinvariant verbleibenden Messteilen (20, 30), stillsetzt.

2. Rotationsrheometer nach Anspruch 1, **dadurch gekennzeichnet, dass** die Kontaktteile (31, 41) des Schaltkontakts (40) in einer Richtung (R') relativ zueinander bewegbar angeordnet sind, die parallel zur Richtung (R) der relativen Bewegung der Messteile (20, 30) im Zuge ihrer Verstellung verläuft oder eine parallel zu dieser Bewegungsrichtung (R) verlaufende Komponente besitzt.

3. Rotationsrheometer nach Anspruch 1 oder 2, **dadurch gekennzeichnet, dass** die beiden Kontaktteile (31, 41) des jeweiligen Schaltkontakts (40) jeweils auf einem von zwei relativ zueinander bewegbaren bzw. voneinander baulich eigenständigen bzw. trennbaren Bauteilen (I, II, III) des Kraftkreises (K) des Rotationsrheometers liegen und dass zumindest eine Federeinheit (50) vorgesehen ist, mit der diese beiden Bauteile (I, II, III) und die Kontakte (31, 41) des Schaltkontakts (40) durch Federkraft aneinander oder aufeinander zu gedrückt und in dieser Lage gehalten sind.

4. Rotationsrheometer nach einem der Ansprüche 1 bis 3, **dadurch gekennzeichnet, dass** der Schaltkontakt (40) auf einzelnen Komponenten des Kraftkreises (K) des Rotationsrheometers, vorzugsweise einem Träger (10) oder einer Tragplatte des Mess- bzw. Antriebsmotors (M), im Verlauf einer Messwelle (22), im Verlauf einer Antriebsspindel (23) der Verstelleinrichtung (1) oder im Verlauf der Halterung für den Mess- oder Antriebsmotor (M) oder jeweils zwischen den einzelnen Komponenten oder im Verbindungs- bzw. Übergangsbereich von einer Komponente zur anderen angeordnet sind.

5. Rotationsrheometer nach einem der Ansprüche 1 bis 4, **dadurch gekennzeichnet, dass** der Kraftkreis (K) des Rotationsrheometers als Komponenten ein Stativ (32), ein Gestell (33), einen Träger (10) für den Antriebs- oder Messmotor (M) sowie den Messteil (20) und die Messwelle (22), und die Verstelleinrichtung (1) zur Höhenverstellung des Messteils (20) bzw. die Spindel (23) und/oder deren Antrieb (34) umfasst.

6. Rotationsrheometer nach einem der Ansprüche 1 bis 5, **dadurch gekennzeichnet, dass** die für die Abschaltung oder Stillsetzung der Verstelleinrichtung (1) zusammenwirkenden, gegebenenfalls als Kontaktflächen ausgebildeten, Kontaktteile (31, 41) des Schaltkontakts (40) auf physisch trennbaren und unabhängig voneinander bewegbaren Bauteilen (I, II, III) bzw. Komponenten des Kraftkreises (K) liegen, welche in der Richtung (R), in der der Messspalt (S) veränderlich und verstellbar ist bzw. in der die Messteile (20, 30) aufeinander zu bzw. voneinander weg bewegbar sind bzw. insbesondere parallel zu der in der Probe auftretenden und gegebenenfalls gemessenen Komponente der Normalkraft, mit Federkraft gegeneinander gedrückt sind.

7. Rotationsrheometer nach einem der Ansprüche 1 bis 6, **dadurch gekennzeichnet, dass** die Verstelleinrichtung (1) bei Öffnung des Schaltkontakts (40) die Verstellbewegung der Messteile (20, 30) relativ zueinander abrupt abbricht.

8. Rotationsrheometer nach einem der Ansprüche 1 bis 7, wobei auf der Verstelleinrichtung (1) ein Träger (10), vorzugsweise mit einer Spindel (23), höhenverstellbar gelagert ist, der den Antriebs- und Messmotor (M) sowie die Messwelle (22) des oben liegendes Messteils (20) trägt, **dadurch gekennzeichnet, dass** der Träger (10) zumindest mit zwei Bauteilen (I, II, III) gebildet ist, wobei die jeweiligen im Kraftkreis (K) aufeinanderfolgenden Bauteile (I, II; II, III) jeweils mit zumindest einer Federeinheit (50) mit vorgegebener Federkraft aneinander gedrückt sind und dass bei Einwirken einer die Federkraft übersteigenden Trennkraft auf die Bauteile (I, II; II, III) die beiden aneinander angedrückten Bauteile (I, II; II, III) voneinander entfernbar sind und gleichzeitig damit die Kontaktteile (31, 41) des Schaltkontakts (40) voneinander trennbar sind.

9. Rotationsrheometer nach einem der Ansprüche 1 bis 8, wobei die Verstelleinrichtung (1) eine auf einer Antriebsspindel (23) höhenverstellbar gelagerte Mutter (2) trägt, **dadurch gekennzeichnet, dass** die Mutter (2) einen, vorzugsweise ringförmigen, Kontaktbauteil (12) mit einem nach oben ragenden Kontaktteil (31) trägt, wobei oberhalb des Kontaktteils (31) ein vorzugsweise von einem Kontaktring gebildeter Kontaktteil (41) liegt, der mit einer Spannhülse (5) verbunden ist, die in ihrem unteren Ende einen Spannring (6) trägt, wobei zwischen dem Spannring (6) und dem Träger (10) des Antriebs- oder Messmotors (M) bzw. der Messwelle (22) eine Schraubenfeder (7) mit vorgegebener Federkraft angeordnet ist und zwischen der Mutter (2) und dem Spannring (6) eine weitere Schraubenfeder (11) mit vorgegebener Federkraft angeordnet ist.

10. Rotationsrheometer nach einem der Ansprüche 1 bis 9, wobei die Verstelleinrichtung (1) eine auf einer Antriebsspindel (23) höhenverstellbar gelagerte Mutter (2) trägt, **dadurch gekennzeichnet, dass** der Träger (10) einen nach oben ragenden Kontaktteil (31) trägt, dass oberhalb des Trägers (10) ein Kontaktbauteil (12) mit einem nach unten ragenden Kontaktteil (41) angeordnet ist, der von oben her an den vom Träger (10) getragenen Kontaktteil (31) anlegbar ist, welcher Kontaktteil (31) von einer Spannhülse (5) getragen ist, die in ihrem unteren Ende einen Spannring (6) trägt, wobei zwischen dem Spannring (6) und dem Träger (10) des Antriebs- oder Messmotors (M) bzw. der Messwelle (22) eine Schraubenfeder (7) mit vorgegebener Federkraft angeordnet ist und zwischen der Mutter (2) und dem Spannring (6) eine weitere Schraubenfeder (11) mit vorgegebener Federkraft angeordnet ist.

11. Rotationsrheometer nach Anspruch 9 oder 10, **dadurch gekennzeichnet, dass** die Schraubenfeder (7) die weitere Schraubenfeder (11) umgibt und gegebenenfalls unterhalb des Schaltkontakts (40) verläuft und/oder dass die Spannhülse (5) zwischen der Schraubenfeder (7) und der weiteren Schraubenfeder (11) liegt und/oder dass die weitere Schraubenfeder (11) die Spindel (23) umgibt.

12. Rotationsrheometer nach einem der Ansprüche 1 bis 11, **dadurch gekennzeichnet, dass** die Kontaktteile (31, 41) mit Blatt- oder Schrauben- oder Spiralfedern belastet sind, deren Federkraft in Richtung (R) der Öffnungs- und Schließbewegung der Kontaktteile (31, 41) wirkt oder eine in dieser Richtung (R) wirkende Federkraftkomponente besitzt.

13. Rotationsrheometer nach einem der Ansprüche 1 bis 12, **dadurch gekennzeichnet, dass** zur Detektion der von zumindest einem Messteil (20, 30) im Zuge seiner Verstellung durch eine von der Probe ausgeübte Kraft zusätzlich zu den auf den Kraftfluss im Kraftkreis (K) ansprechenden Schalteinheit (40) bzw. Schaltkontakten (31, 41) eine Einrichtung zur Messung der auftretenden, von der Probe ausgeübten positiv oder negativ zur Richtung des Kraftflusses gerichteten Normalkraft F_{N} vorgesehen ist.

14. Rotationsrheometer nach einem der Ansprüche 9 oder 10, **dadurch gekennzeichnet, dass** die als Hubfeder wirkende Schraubenfeder (7) und die als Senkfeder wirkende weitere Schraubenfeder (11) jeweils mit einer Vorspannungskraft bzw. einem Grenzkraftwert vorgespannt sind, die bzw. der dem Gewicht der zu tragenden Komponenten bzw. Apparatur des Rotationsrheometers und einem vorgegebenen Kraftwert entspricht, wobei dieser vorgegebene Kraftwert einem von der Probe aus auf den Messteil (20) in positiver und negativer Richtung des Kraftflusses ausgeübten und noch als tolerierbar angesehenen Kraftwert entspricht.

15. Rotationsrheometer nach einem der Ansprüche 3 bis 14, **dadurch gekennzeichnet, dass** die Kontaktteile (31, 41) von Teilen der die Bauteile (I, II, III) belastenden Federeinheiten (50) gebildet sind.

## Claims

1. A rotational rheometer comprising two measurement parts (20, 30) delimiting the measurement gap (S) and movable relative to one another in a predetermined movement direction (R) by an adjustment device (1),
**characterised in that** at least one switching unit, responding to changes in the force flow in the force circuit (K) of the rotational rheometer delimited by the measurement parts (20, 30) and having contact parts (31, 41) comprising switching contacts (40) for activation of the adjustment device (1), is provided and stops said adjustment device (1) when a predetermined limit force value is exceeded for the force flow in a positive direction, possibly also without relative movement of the measurement parts, or with the measurement parts (20, 30) remaining in the same position,
and stops the adjustment device (1) when a predetermined limit force value is exceeded for the force flow in a negative direction, possibly also without relative movement of the measurement parts, or with the measurement parts (20, 30) remaining in the same position.

2. The rotational rheometer according to claim 1, **characterised in that** the contact parts (31, 41) of the switching contact (40) are disposed movably relative to one another in a direction (R') which extends parallel to the direction (R) of the relative movement of the measurement parts (20, 30) during the course of their adjustment or has a component running parallel to this direction of movement (R).

3. The rotational rheometer according to claim 1 or 2, **characterised in that** the two contact parts (31, 41) of each switching contact (40) lie in each case on one of two members (I, II, III) of the force circuit (K) of the rotational rheometer which are movable relative to one another or are structurally independent of one another or can be separated from one another, and **in that** at least one spring unit (50) is provided, with which said two members (I, II, III) and the contacts (31, 41) of the switching contact (40) are pressed onto one another or toward one another by spring force and are held in this position.

4. The rotational rheometer according to any one of claims 1 to 3, **characterised in that** the switching contacts (40) are disposed on individual components of the force circuit (K) of the rotational rheometer, preferably a carrier (10) or a carrier plate of the measurement or drive motor (M), along a measurement shaft (22), along a drive spindle (23) of the adjustment device (1), or along the holder for the measurement or drive motor (M), or between the individual components or in a connection or transition region from one component to the other.

5. The rotational rheometer according to any one of claims 1 to 4, **characterised in** the force circuit (K) of the rotational rheometer comprises, as components, a stand (32), a frame (33), a carrier (10) for the drive or measurement motor (M) and the measurement part (20) and the measurement shaft (22), and the adjustment device (1) for adjusting the height of the measurement part (20), or the spindle (23) and/or the drive (34) thereof.

6. The rotational rheometer according to any one of claims 1 to 5, **characterised in that** the contact parts (31, 41) of the switching contact (40), possibly formed as contact faces and interacting for switching off or stopping of the adjustment device (1), lie on physically separable and independently movable members (I, II, III) or components of the force circuit (K), which are pressed against one another with spring force, in particular parallel to the component of the normal force occurring in the sample and optionally measured, in the direction (R) in which the measurement gap (S) is variable and adjustable, or in which the measurement parts (20, 30) are movable toward one another or away from one another.

7. The rotational rheometer according to any one of claims 1 to 6, **characterised in that** the adjustment device (1) abruptly terminates the adjustment movement of the measurement parts (20, 30) relative to one another when the switching contact (40) is opened.

8. The rotational rheometer according to any one of claims 1 to 7, wherein a carrier (10), preferably with a spindle (23), is mounted height-adjustably on the adjustment device (1) and carries the drive and measurement motor (M) and the measurement shaft (22) of the measurement part (20) lying above, **characterised in that** the carrier (10) is formed at least with two members (I, II, III), wherein the members (I, II; II, III) lying in each case successively in the force circuit (K) are pressed against one another in each case by at least one spring unit (50) with a predetermined spring force, and **in that** when a separating force exceeding the spring force acts on the members (I, II; II, III), the two members (I, II; II, III) pressed against one another can be removed from one another and at a same time said contact parts (31, 41) of the switching contact (40) can be separated from one another.

9. The rotational rheometer according to any one of claims 1 to 8, wherein the adjustment device (1) carries a nut (2) mounted height-adjustably on a drive spindle (23), **characterised in that** the nut (2) carries a preferably annular contact member (12) having an upwardly protruding contact part (31), wherein a contact part (41) preferably formed by a contact ring lies above the contact part (31) and is connected to a clamping sleeve (5) which carries a clamping ring (6) at its lower end, wherein a coil spring (7) with predetermined spring force is disposed between the clamping ring (6) and the carrier (10) of the drive or measurement motor (M) or the measurement shaft (22) and a further coil spring (11) with predetermined spring force is disposed between the nut (2) and the clamping ring (6).

10. The rotational rheometer according to any one of claims 1 to 9, wherein the adjustment device (1) carries a nut (2) mounted height-adjustably on a drive spindle (23), **characterised in that** the carrier (10) carries an upwardly projecting contact part (31), **in that** a contact member (12) with a downwardly projecting contact part (41) is arranged above the carrier (10) and can be applied from above onto the contact part (31) carried by the carrier (10), which contact part (31) is carried by a clamping sleeve (5) which carries a clamping ring (6) at its lower end, wherein a coil spring (7) with predetermined spring force is disposed between the clamping ring (6) and the carrier (10) of the drive or measurement motor (M) or of the measurement shaft (22), and a further coil spring (11) with a predetermined spring force is disposed between the nut (2) and the clamping ring (6).

11. The rotational rheometer according to claim 9 or 10, **characterised in that** the coil spring (7) surrounds the further coil spring (11) and optionally runs beneath the switching contact (40), and/or **in that** the clamping sleeve (5) lies between the coil spring (7) and the further coil spring (11), and/or **in that** the further coil spring (11) surrounds the spindle (23).

12. The rotational rheometer according to any one of claims 1 to 11, **characterised in that** the contact parts (31, 41) are loaded by leaf springs or coil springs or helical springs, the spring force of which acts in the direction (R) of the opening and closing movement of the contact parts (31, 41) or has a spring force component acting in this direction (R).

13. The rotational rheometer according to any one of claims 1 to 12, **characterised in that** in order to detect at least one of the measurement parts (20, 30), during the course of adjustment thereof, by a force exerted by the sample, in addition to the switching unit (40) or switching contacts (31, 41) responding to the force flow in the force circuit (K), a device for measuring the normal force F_{N} exerted by the sample and directed positively or negatively with respect to the direction of the force flow is provided.

14. The rotational rheometer according to one of claims 9 or 10, **characterised in that** the coil spring (7) acting as lifting spring and the further coil spring (11) acting as lowering spring are each pre-stressed by a pre-stressing force or a limit force value corresponding to the weight of the components or apparatus of the rotational rheometer to be carried and a predetermined force value, wherein this predetermined force value corresponds to a force value exerted by the sample onto the measurement part (20) in positive and negative directions of the force flow and still regarded as tolerable.

15. The rotational rheometer according to any one of claims 3 to 14, **characterised in that** the contact parts (31, 41) are formed by parts of the spring units (50) loading the members (I, II, III).

## Revendications

1. Rhéomètre rotatif comprenant deux parties de mesure (20, 30) limitant la fente de mesure (S), pouvant se déplacer l'une par rapport à l'autre dans une direction de déplacement prédéfinie (R) à l'aide d'un dispositif de déplacement (1),
**caractérisé en ce qu'**il est prévu au moins une unité de commutation répondant aux modifications dans le flux de force dans le circuit de forces (K) du rhéomètre rotatif limité par les parties de mesure (20, 30), qui comprend des contacts de commutation (40) présentant des parties de contact (31, 41) pour l'activité du dispositif de déplacement (1), qui arrête le dispositif de déplacement (1) en cas de dépassement d'une valeur de force limite prédéfinie pour le flux de force dans la direction positive, éventuellement également sans mouvement relatif des parties de mesure ou au cas où les parties de mesure (20, 30) demeurent dans une position invariante
et qui arrête le dispositif de déplacement (1) en cas de dépassement d'une valeur de force limite prédéfinie pour le flux de force dans la direction négative, éventuellement également sans mouvement relatif des parties de mesure ou au cas où les parties de mesure (20, 30) demeurent dans une position invariante.

2. Rhéomètre rotatif selon la revendication 1, **caractérisé en ce que** les parties de contact (31, 41) du contact de commutation (40) sont montées mobiles l'une par rapport à l'autre dans une direction (R') qui est parallèle à la direction (R) du mouvement relatif des parties de mesure (20, 30) au cours de leur déplacement ou qui possède un composant parallèle à ladite direction de déplacement (R).

3. Rhéomètre rotatif selon la revendication 1 ou 2, **caractérisé en ce que** les deux parties de contact (31, 41) du contact de commutation respectif (40) se situent respectivement sur l'un des deux éléments (I, II, III) pouvant se déplacer l'un par rapport à l'autre ou individuels structuralement ou séparables, du circuit de force (K) du rhéomètre rotatif et **en ce qu'**il est prévu au moins une unité de ressorts (50) qui permet auxdits deux éléments (I, II, III) et aux contacts (31, 41) du contact de commutation (40) de s'appuyer l'un contre l'autre ou l'un sur l'autre et de se maintenir dans cette position.

4. Rhéomètre rotatif selon l'une des revendications 1 à 3, **caractérisé en ce que** le contact de commutation (40) est disposé sur chacun des composants du circuit de forces (K) du rhéomètre rotatif, de préférence sur un support (10) ou une plaque de support du moteur d'entraînement ou de mesure (M), sur le tracé d'un arbre de mesure (22), sur le tracé d'une broche d'entraînement (23) du dispositif de déplacement (1) ou sur le tracé de l'élément de retenue pour le moteur d'entraînement ou de mesure (M) ou respectivement entre les composants individuels ou dans la zone de transition ou de liaison d'un composant à un autre.

5. Rhéomètre rotatif selon l'une des revendications 1 à 4, **caractérisé en ce que** le circuit de forces (K) du rhéomètre rotatif comprend en tant que composants, un pied (32), un châssis (33), un support (10) pour le moteur de mesure ou d'entraînement (M) et pour la partie de mesure (20) et l'arbre de mesure (22) et le dispositif de déplacement (1) servant à régler la hauteur de la parie de mesure (20) ou de la broche (23) et/ou leur entraînement (34).

6. Rhéomètre rotatif selon l'une des revendications 1 à 5, **caractérisé en ce que** les parties de contact (31, 41) du contact de commutation (40) conçues éventuellement sous la forme de surfaces de contact, coopérant pour l'arrêt ou l'interruption du dispositif de déplacement (1), se trouvent sur des éléments (I, II, III) mobiles indépendamment les uns des autres et physiquement séparables ou des composants du circuit de forces (K), qui, dans la direction (R), dans laquelle la fente de mesure (S) est modifiable ou réglable ou dans laquelle les parties de mesure (20, 30) peuvent se rapprocher l'une de l'autre ou s'éloigner l'une de l'autre, en particulier parallèlement au composant de la force normale éventuellement mesuré et apparaissant dans la sonde, sont pressés l'une contre l'autre par la force de ressort.

7. Rhéomètre rotatif selon l'une des revendications 1 à 6, **caractérisé en ce que** le dispositif de déplacement (1) interrompt brusquement le mouvement de déplacement des parties de mesure (20, 30) l'une par rapport à l'autre au moment de l'ouverture du contact de commutation (40).

8. Rhéomètre rotatif selon l'une des revendications 1 à 7, sur le dispositif de déplacement (1) étant monté réglable en hauteur un support (10), de préférence avec une broche (23), qui porte le moteur de mesure et d'entraînement (M) ainsi que l'arbre de mesure (22) de la partie de mesure supérieure (20), **caractérisé en ce que** le support (10) est constitué d'au moins deux éléments (I, II, III), chacun des éléments (I, II, III) consécutifs dans le circuit de forces (K) étant pressés respectivement au moyen d'au moins une unité de ressorts (50) les uns contre les autres par l'action d'une force de ressort prédéfinie et **en ce que** par l'action d'une force de séparation dépassant la force de ressort sur les éléments (I, II, III), les deux éléments (I, II, III) pressés l'un contre l'autre s'éloignent l'un de l'autre et simultanément les parties de contact (31, 41) du contact de commutation (40) se séparent par conséquent l'un de l'autre.

9. Rhéomètre rotatif selon l'une des revendications 1 à 8, le dispositif de déplacement (1) supportant un écrou (2) réglable en hauteur monté sur une broche d'entraînement (23), **caractérisé en ce que** l'écrou (2) supporte un élément de contact (12) de préférence annulaire doté d'une partie de contact (31) faisant saillie vers le haut, au-dessus de la partie de contact (31) se trouvant une partie de contact (41) formée de préférence à partir d'un anneau de contact, qui est reliée à une douille de serrage (5), qui supporte sur son extrémité inférieure une bague de serrage (6), entre la bague de serrage (6) et le support (10) du moteur de mesure ou d'entraînement (M) ou de l'arbre de mesure (22) étant disposé un ressort hélicoïdal (7) doté de la force de ressort prédéfinie et entre l'écrou (2) et la bague de serrage (6) étant disposé un autre ressort hélicoïdal (11) doté de la force de ressort prédéfinie.

10. Rhéomètre rotatif selon l'une des revendications 1 à 9, le dispositif de déplacement (1) supportant un écrou (2) réglable en hauteur monté sur une broche d'entraînement (23), **caractérisé en ce que** le support (10) supporte une partie de contact (31) faisant saillie vers le haut, **en ce qu'**au-dessus du support (10) est disposé un élément de contact (12) dont une partie de contact (41) fait saillie vers le bas, qui peut être placé à partir du haut sur la partie de contact (31) supportée par le support (10), ladite partie de contact (31) est supportée par une douille de serrage (5) qui supporte sur son extrémité inférieure une bague de serrage (6), entre la bague de serrage (6) et le support (10) du moteur de mesure ou d'entraînement (M) ou de l'arbre de mesure (22) étant disposé un ressort hélicoïdal (7) doté de la force de ressort prédéfinie et entre l'écrou (2) et la bague de serrage (6) étant disposé un autre ressort hélicoïdal (11) doté de la force de ressort prédéfinie.

11. Rhéomètre rotatif selon la revendication 9 ou 10, **caractérisé en ce que** le ressort hélicoïdal (7) entoure l'autre ressort hélicoïdal (11) et s'étend éventuellement en dessous du contact de commutation (40) et/ou **en ce que** la douille de serrage (5) se situe entre le ressort hélicoïdal (7) et l'autre ressort hélicoïdal (11) et/ou **en ce que** l'autre ressort hélicoïdal (11) entoure la broche (23).

12. Rhéomètre rotatif selon l'une des revendications 1 à 11, **caractérisé en ce que** les parties de contact (31, 41) sont sollicitées par des ressorts spiral ou hélicoïdaux ou à lames, dont la force de ressort agit dans la direction (R) du mouvement de fermeture et d'ouverture des parties de contact (31, 41) ou possède un composant de force de ressort agissant dans ladite direction (R).

13. Rhéomètre rotatif selon l'une des revendications 1 à 12, **caractérisé en ce que** pour la détection d'au moins une partie de mesure (20, 30) au cours de son déplacement par la force exercée par la sonde en plus des contacts de commutation (31, 41) ou de l'unité de commutation (40) en réponse au flux de forces dans le circuit de forces (K), il est prévu un dispositif de mesure de la force normale F_{N} apparaissant, orientée positivement ou négativement dans la direction du flux de forces, exercée par la sonde.

14. Rhéomètre rotatif selon l'une des revendications 9 ou 10, **caractérisé en ce que** les ressort hélicoïdaux (7) agissant comme ressorts de levage et les autres ressorts hélicoïdaux (11) agissant comme ressorts d'abaissement sont respectivement précontraints par une force de précontrainte ou une valeur de force limite, qui correspond(ent) au poids des composants à supporter ou à l'appareil du rhéomètre rotatif et à la valeur de force prédéfinie, ladite valeur de force prédéfinie correspondant à la valeur de force considérée comme tolérable et exercée par la sonde sur la partie de mesure (20) dans la direction positive et négative du flux de forces.

15. Rhéomètre rotatif selon l'une des revendications 3 à 14, **caractérisé en ce que** les parties de contact (31, 41) sont constituées par des parties des unités de ressorts (50) sollicitant les éléments (I, II, III).
